# EUROPEAN PATENT APPLICATION

(11) **EP 4 837 961 A1**
(43) Date of publication of application: **11.11.2026**
(21) Application number: 25736260.8
(22) Date of filing: 02.01.2025
(51) Int. Cl.: C12N 5/071, G01N 33/50

(54) **NOVEL ORGANOID CULTURE SYSTEM FOR ASSESSING TOXICITY OF NANOMATERIALS**

(30) Priority: 03.01.2024 KR 20240000794
(71) Applicant: Korea Research Institute of Standards and Science, Daejeon 34113 (KR)
(72) Inventor: BAEK, Ah Ruem, Daejeon 34118 (KR); HEO, Min Beom, Daejeon 34032 (KR); LEE, Tae Geol, Daejeon 34130 (KR)
(74) Representative: Ipsilon
(86) International application number: PCT/KR2025/000057
(87) International publication number: WO 2025/147106

(57) **Abstract**

The present invention relates to a liver organoid suspension culture method for assessing the safety and toxicity of nanomaterials and, more specifically, to a culture method in which nanomaterials can be introduced into cells constituting an organoid, wherein when a suspension culture system according to the present invention is used, the organoid culture method provides a reliable tool for assessing the stability of nanomaterials, is highly likely to be applicable to high-throughput nano safety screening, and can accelerate the research and development of nanomaterials in various fields.

## Description

### Technical Field

The present invention relates to a method for the floating culture of organoids for assessing the safety and toxicity of nanomaterials, and more specifically, to a culture method in which nanomaterials may be introduced into the cells of organoids.

### Background Art

The discovery of numerous nanomaterials has expanded into new dimensions accompanied by the rapid development of nanotechnology. The range of potential applications of nanomaterials is diverse, from cosmetics and biomedicines to nano-electronics and mechanical engineering. Consequently, the development of nanotechnology has led to a notable increase in public exposure to nanomaterials. This rapid expansion of nanotechnologies for human use is limited by a lack of understanding of how the unique physico-chemical properties of these materials influence biological systems, particularly, toxicity mechanisms. As suggested in another study, it is primordial to know the range of health and safety hazards related to nanomaterial exposure to advance knowledge and accelerate the development of nanotechnology.

Two-dimensional (2D) cell-based assays have been regarded as the standard method for *in vitro* nano-toxicity assessment owing to their cost-effectiveness, reproducibility, and high throughput, despite a lack of reliability between *in vitro* and *in vivo*. Animal models are the gold standard in toxicological studies; however, *in vivo* experiments are high-cost, low-throughput, time-consuming, labor-intensive, and accompanied by ethical constraints. Moreover, the Environmental Protection Agency (EPA) announced a plan to eliminate mammalian toxicity testing by 2035. Organoids, which are self-organized, multi-cellular 3D tissue, are considered promising models as an alternative to animal models. Organoid models have some benefits in terms of having a comparatively longer lifespan than 2D cell models and being completely devoid of the ethical issues associated with animal testing. In addition, organoids exhibit high similarity to their actual organ by having structural and functional properties of the organ. The significant cellular heterogeneity of organoids replicates the architectural barriers and complexity of real organs.

Recently, several studies have shown the potential of organoids as a physiologically relevant model to assess nanomaterial toxicity. Recent studies reported the toxicity of silver nanoparticles using cerebral organoids, and established a toxicity assessment of nanoparticles using colon organoids. Although these studies shed light on the potential of organoids as a tool for assessing nanoparticle toxicity, limitations still exist. One major limitation is the lack of confirmation of nanoparticle internalization in cells within organoids. Despite these studies reporting the toxicity of nanoparticles in organoid systems, these studies do not provide direct evidence of nanoparticle internalization and subcellular localization. This lack of confirmation raises concerns regarding the reliability and validity of the reported toxicological outcomes.

Another limitation of organoid-based toxicity assessments of nanoparticles is the lack of standardization in the preparation and handling of the organoids. The extracellular matrix (ECM), a unique meshwork composed of structural proteins and glycosaminoglycans, is used to support the physical configuration for 3D morphogenesis and growth of organoids. Organoids are typically embedded and cultured in solidified ECM scaffolds to form a dome shape. However, organoids that have been grown in an ECM dome show size heterogeneity induced by the spatiotemporal gradient of morphogens, oxygen, and nutrient which is unsuitable for *in vitro* screening assays. Furthermore, nanoparticles aggregate in ECM and exhibit inefficient cellular internalization owing to the properties of the ECM, including its complex network and charged components.

The present invention reports the establishment of a new organoid culture system for assessing the safety and toxicity of nanomaterials. The present invention shows that the nanomaterials are internalized within organoids cultured by the culture system, overcoming the limitations associated with the ECM, a barrier of nanomaterials to nanomaterial accumulation. The protocol of the present invention produces relatively uniformly sized organoids and enables the manipulation of the number of organoids, suitable for standardization. The results of the present invention suggest that the floating culture of organoids provides an accurate and reliable platform for the safety assessment of nanomaterials.

### DISCLOSURE

### Technical Problem

The present invention relates to the establishment of a new organoid culture system for safety and toxicity assessments of nanomaterials.

An object of the present invention is to overcome the limitations associated with the extracellular matrix (ECM), a barrier of nanomaterials to nanomaterial accumulation, and provide an accurate and reliable platform for the safety assessment of nanomaterials by producing relatively uniformly sized organoids and manipulating the number of organoids suitable for standardization, thereby providing a toxicity assessment specific to each tissue or individual.

### Technical Solution

The present invention relates to a novel organoid culture method for assessing the safety and toxicity of nanomaterials, and an object of the present invention is to overcome the limitations associated with the ECM by using organoids obtained by a floating culture method rather than existing adherent organoids, and establish an accurate and reliable platform by producing relatively uniformly sized organoids and manipulating the number of organoids suitable for standardization.

In one embodiment of the present invention, the nanomaterials may be nanoparticles that generate toxicity when reacting with biological tissue, and specifically may be metal nanomaterials, heavy metal nanomaterials, inorganic oxide nanomaterials, carbon-based nanomaterials, or polymer nanomaterials, without being limited thereto.

In one embodiment of the present invention, the nanomaterials are capable of causing changes in biological metabolism and immune responses when reacting with biological tissue, and specifically, may cause metabolic disorders, recover metabolic disorders, induce inflammatory responses, alleviate inflammatory responses, induce cancer, or ameliorate cancer, without being limited thereto.

In one embodiment of the present invention, the organoids may be formed through self-renewal, differentiation, or self-organization of stem cells selected from the group consisting of adult stem cells, embryonic stem cells, and induced pluripotent stem cells.

In one embodiment of the present invention, the organoids may mimic the function of the cerebrum, gastrointestinal tract, intestine (small intestine, large intestine), stomach, lingua, thyroid gland, thymus, testis, prostate, liver, pancreas, epithelium, kidney, heart, retina, glioblastoma, etc., without being limited thereto.

### Advantageous Effects

The organoid culture method according to the present invention may offer efficient assays for nanomaterial-induced toxicity assessment.

The organoid culture method according to the present invention may perform a more accurate toxicity assessment of nanomaterials by using the entire organoids, because the organoids are in a floating state, unlike adherent organoids in which only a portion of the organoids is used

The organoid culture method according to the present invention may exhibit uniform sizing and structural integrity and may be more conducive to the internalization of nanoparticles, thereby performing a more accurate toxicity assessment of nanomaterials compared to conventional organoid toxicity assessment platforms.

### Brief Description of Drawings

FIG. 1 shows dark-field microscopy images of ECM dome following exposure to gold nanoparticles (AuNPs). FIGS. 2 and 3 below, including FIG. 1, show the limitations of the existing extracellular matrix (ECM)-embedded organoid culture system for safety assessment of nanomaterials.
FIG. 2 shows representative bright-field microscopy images of mouse liver organoids (hereinafter referred to as mLOs) embedded and cultured in domes containing various concentrations of ECM.
FIG. 3 shows dark-field microscopy images of ECM-embedded mLOs after exposure to AuNPs.
FIG. 4 relates to the preparation and optimization of a floating culture or ganoid system, and schematically illustrates the floating culture system for mLOs.
FIG. 5 relates to the optimization process of the floating organoid culture conditions. Specifically, FIG. 5A shows the results of microscopic observation after mLOs embedded and cultured in an ECM dome for 3 days were subjected to floating culture in adherent cell culture plates or ultra-low attachment plates, and FIG. 5B shows the results of monitoring using a live-cell analysis system after ECM-embedded mLOs were cultured for 3 to 5 days and then subjected to floating culture.
FIG. 6 shows fluorescence microscopy images of ECM-embedded mLOs and floating mLOs immunostained with differentiation marker proteins to confirm differentiation.
FIG. 7A shows the results of analyzing gene expression in undifferentiated mLOs, differentiated ECM-embedded mLOs, and floating mLOs by qRT-PCR, and FIG. 7B is a heat map illustrating the result of RNAseq analysis.
FIG. 8 shows variation in size and size distribution of mLOs in ECM-embedded and floating culture organoid systems. FIG. 8A shows the automatic detection and quantification of mLOs, and FIG. 8B is a graph showing the size distribution of mLOs based on diameters and counts of organoids recognized in FIG. 8A.
FIG. 9 shows the results of comparing the introduction of nanomateriaLS into liver organoids according to a cell culture method. FIG. 9A shows fluorescence microscopy images of ECM-embedded and floating culture organoids following exposure to FITC-polystyrene, and FIG. 9B shows fluorescence microscopy images of paraffin sections of organoids shown in FIG. 9A.
FIG. 10 shows the results of flow cytometry analysis of ECM-embedded and floating culture organoids following exposure to FITC-polystyrene.
FIG. 11A shows dark-field microscopy images of organoids after exposure to AuNPs, and FIG. 11B shows dark-field microscopy images of floating culture organoids over time after exposure to AuNPs.
FIG. 12 shows the results of evaluating the cytotoxicity of nanoparticles in mLOs in extracellular matrix (ECM)-embedded and floating culture systems. It shows fluorescence microscopy images of calcein/propidium iodide (PI)-stained mLOs following exposure to AuNPs and ZnO NPs.
FIG. 13A is a bar graph showing the live/dead ratio obtained by quantifying the fluorescence intensity of calcein/propidium iodide shown in FIG. 12, and FIG. 13B shows the results of flow cytometry-based assay of cell death.

### Best Mode

The present invention relates to a novel organoid culture method for assessing the safety and toxicity of nanomaterials, and an object of the present invention is to overcome the limitations associated with the ECM by using organoids obtained by a floating culture method rather than existing ECM-embedded organoids, and establish an accurate and reliable platform by producing uniformly sized organoids and manipulating the number of organoids suitable for standardization.

In one embodiment of the present invention, the nanomaterials may be nanoparticles that generate toxicity when reacting with biological tissue, and specifically may be metal nanomaterials, heavy metal nanomaterials, inorganic oxide nanomaterials, carbon-based nanomaterials, or polymer nanomaterials, without being limited thereto.

In one embodiment of the present invention, the nanomaterials may be metal nanomaterials, heavy metal nanomaterials, inorganic oxide nanomaterials, carbon-based nanomaterials, polymer nanomaterials, or the like. Specifically, the nanomaterials may be polystyrene nanoparticles (PS NPs), gold nanoparticles (Au NPs), zinc oxide nanoparticle (ZnO NPs), fine dust, chemical nanomaterials, or cosmetic compositions. More specifically, the nanomaterials may be nanomaterials such as PS NPs, Au NPs, or ZnO NPs, without being limited thereto.

In one embodiment of the present invention, the nanomaterials are capable of causing changes in biological metabolism and immune responses when reacting with biological tissue, and specifically, may cause metabolic disorders, recover metabolic disorders, induce inflammatory responses, alleviate inflammatory responses, induce cancer, or ameliorate cancer, without being limited thereto.

In one embodiment of the present invention, the nanomaterials may be those that may cause inflammatory responses, immune responses, metabolic diseases, or cancer, without being limited thereto.

In the present invention, organoids are three-dimensional cell aggregates formed through self-renewal and self-organization from adult stem cells, embryonic stem cells, or induced pluripotent stem cells, and contain model organic-specific cells. They can similarly reproduce the physiological functions of the human body. By establishing organoids from patient tissues, they enable disease modeling and drug screening based on genetic information, and are attracting attention as an alternative to gene therapy and organ transplantation.

In one embodiment of the present invention, the organoids may be formed through self-renewal, differentiation, or self-organization of stem cells selected from the group consisting of adult stem cells, embryonic stem cells, and induced pluripotent stem cells.

In one embodiment of the present invention, the organoids may mimic the function of the cerebrum, gastrointestinal tract, intestine (small intestine, large intestine), stomach, lingua, thyroid gland, thymus, testis, prostate, liver, pancreas, epithelium, kidney, heart, retina, glioblastoma, etc., without being limited thereto.

In one embodiment of the present invention, the ECM-embedded organoid culture is routinely maintained in an organic culture medium, and the organoids may be passaged every 5 to 10 days, specifically every 6 to 8 days, more specifically every 7 days.

In one embodiment of the present invention, a method for producing spherical organoids may be performed using an ECM having a high concentration of 50% or more. Specifically, the ECM may have a concentration of 50 to 100%, more specifically 50% or more.

In one embodiment of the present invention, as a method of dissociating the passaged organoids, mLOs are dissociated by mechanical trituration using a 1-ml syringe equipped with a 26G blunt needle.

The dissociated mLOs are centrifuged, and then the supernatant is removed.

In one embodiment of the present invention, the centrifugation may be performed at 100 to 150 g, specifically of 125 g.

In one embodiment of the present invention, the centrifugation may be performed for 3 to 7 minutes, specifically 4 to 6 minutes, more specifically 5 minutes.

After centrifugation, the pellet is resuspended in 100% Matrigel, and the resulting cell suspension is dispensed in a 48-well plate to form a dome shape. Then, the plate is placed in an incubator at 37°C for 1 hour to solidify the Matrigel.

The Matrigel has a protein structure such as laminin, nidogen, collagen, or heparan sulfate proteoglycan, is composed of a solubilized basement membrane matrix secreted from mouse sarcoma cells, and is used for tissue culture. It may also contain TGF-β and EGF, and may contain various growth factors.

In one embodiment of the present invention, the amount of the cell suspension dispensed into the well plate may vary depending on the size of the plate, but may be 10 to 50 µl, specifically 20 to 30 µl, more specifically 25 µl.

An organoid culture medium is added to the Matrigel solidified in the incubator, and the medium is replaced every 2 or 3 days.

Thereafter, the Matrigel organoids cultured for 3 days are used for floating culture.

An object of the present invention is to assess the toxicity of nanomaterials based on organoids subjected to floating culture.

In one embodiment of the present invention, the method for floating culture of organoids includes: step A) of washing an organoid-containing Matrigel (100%) with PBS; step B) of adding a pre-chilled cell recovery solution to a well, followed by disruption by pipetting; step C) of incubating the organoid-containing Matrigel for about 30 minutes until the Matrigel matrix is fully depolymerized, followed by centrifugation to obtain a pellet; step D) of suspending the pellet in an organoid culture medium containing 5% Matrigel; and step E) of incubating the organoids in an ultra-low attachment plate.

In one embodiment of the present invention, the centrifugation may be performed at 100 to 150 g, specifically of 125 g.

In one embodiment of the present invention, the centrifugation may be performed for 1 to 3 minutes, specifically 1 to 2 minutes.

In one embodiment of the present invention, the Matrigel contained in the organoid culture medium may be contained at a concentration of 1 to 10%, specifically 3 to 8%, more specifically 5%.

In one embodiment of the present invention, the medium during the incubation may be replaced every 1 to 5 days, specifically every 2 to 4 days, more specifically every 2 or 3 days.

Then, the medium is changed to a differentiation medium to differentiate the organoids subjected to floating culture, and the medium is continuously replaced until differentiation is complete.

The composition of the differentiation medium may comprise 10 mM HEPES, 2 mM Glutamax, 1 mM N-acetylcystein, 1% penicillin/streptomycin, 1x B-27, 50 ng/ml EGF (epidermal growth factor), 100 ng/ml FGF10 (fibroblast growth factor), 10 nM recombinant human [Leu15]-gastrin I, 50 nM A83-01, and 10 µM DAPT, without being limited thereto, and may vary depending on the type of organoid.

In one embodiment of the present invention, the differentiation medium may be replaced daily, and the organoids may be incubated with the differentiation medium while replacing the differentiation medium daily for up to 10 to 15 days, specifically up to 11 to 13 days, more specifically up to 12 days, before addition of an anti-inflammatory agent.

In one embodiment of the present invention, the anti-inflammatory agent may be at least one selected from the group consisting of steroids, non-steroids, immune selective anti-inflammatory derivatives, herbal extracts, and antioxidants. Specifically, it may be a steroidal anti-inflammatory agent.

In one embodiment of the present invention, the steroidal anti-inflammatory agent may be prednisone, dexamethasone, triamcinolone, betamethasone, beclomethasone, flunisolide, or fluticasone. Specifically, it may be betamethasone, beclomethasone, or dexamethasone. More specifically, it may be dexamethasone.

In one embodiment of the present invention, the differentiated organoids may be treated with the anti-inflammatory agent for 1 to 5 days, specifically 2 to 4 days, more specifically 3 days.

Using the prepared floating culture organoids, whether the organoids differentiated is analyzed through immunostaining, qRT-PCR, and quantitative analysis such as RNAseq. Based on the analyzed organoids, the cell penetration ability of nanoparticles is analyzed.

In one embodiment of the present invention, the present invention may provide an organoid culture kit for assessing nanomaterial toxicity.

In one embodiment of the present invention, the culture kit may be a kit for culturing organoids using a floating culture method.

In one embodiment of the present invention, the nanomaterials whose toxicity is assessed using the culture kit may be metal nanomaterials, heavy metal nanomaterials, inorganic oxide nanomaterials, carbon-based nanomaterials, or polymer nanomaterials, without being limited thereto.

In one embodiment of the present invention, the nanomaterials whose toxicity is assessed using the culture kit may be metal nanomaterials, heavy metal nanomaterials, inorganic oxide nanomaterials, carbon-based nanomaterials, polymer nanomaterials, or the like. Specifically, the nanomaterials may be polystyrene nanoparticles (PS NPs), gold nanoparticles (Au NPs), zinc oxide nanoparticle (ZnO NPs), fine dust, chemical nanomaterials, or cosmetic compositions. More specifically, the nanomaterials may be nanomaterials such as PS NPs, Au NPs, or ZnO NPs, without being limited thereto.

In one embodiment of the present invention, the organoids that may be used through the kit may be three-dimensional cell aggregates formed through self-renewal and self-organization from adult stem cells, embryonic stem cells, or induced pluripotent stem cells.

In one embodiment of the present invention, the organoids that may be used through the kit have may mimic the function of the cerebrum, gastrointestinal tract, intestine (small intestine, large intestine), stomach, lingua, thyroid gland, thymus, testis, prostate, liver, pancreas, epithelium, kidney, heart, retina, glioblastoma, etc., without being limited thereto.

### Mode for Invention

Hereinafter, one or more embodiments will be described in more detail through examples. However, these examples are intended to illustrate one or more embodiments and the scope of the present invention is not limited to these examples.

### Example 1. Experimental methods and materials

### 1-1. Experimental materials

Collagenase (C9407), DNase I (DN25), [Leu15]-Gastrin 1 (G9145), nicotinamide (N0636), N-acetylcysteine (A0737), PBS (D8537), paraformaldehyde (158127), Triton X-100 (T8787), bovine serum albumin (A9418), calcein-AM (C1430), propidium iodide (P4170), DAPI (D9564) dexamethasone (D4902) and DAPT (D5942) were purchased from Sigma-Aldrich (MO, USA).

Matrigel (356231) and Cell Recovery solution (354253) were acquired from Corning (NY, USA).

High-glucose Dulbecco's modified Eagle's medium (DMEM) (31966-021), Advanced DMEM F/12 (12634-010), B-27 (12587-010), HEPES (15630-056), Glutamax (35050-068), fetal bovine serum (26140-079), penicillin/streptomycin (15140-122), Dispase II (17105-041), and Trypsin/EDTA (25200-056) were purchased from GIBCO (MA, USA). Recombinant murine EGF (315-09), murine Noggin (250-38), and murine HGF (315-23) were obtained from PeproTech (NJ, USA).

A83-01 (2939) was acquired from Tocris Bioscience (Bristol, UK).

Apoptosis detection kit (556547) was purchased from BD Biosciences. TRIzol (15596026) and SYBR Green PCR Master Mix (4309155) were obtained from Thermo Fisher Scientific.

PrimeScript 1st strand cDNA Synthesis Kit (6110A) were purchased from TaKaRa.

FITC-labeled polystyrene nanoparticles (DCFG-L002) were obtained from CD Bioparticles (NY, USA).

AuNPs (EM.GC50) were purchased from BBI solutions (Cardiff, UK).

Zinc oxide (52504965) was purchased from BASF (Ludwigshafen, Germany).

Anti-HNF4α (sc8987) was purchased from Santa Cruz Biotechnology (CA, USA), anti-EpCAM (17-5791-80) was purchased from eBioscience (CA, USA), and fluorescence (Alexa Fluor 488 or 546)-conjugated anti-mouse and rabbit immunoglobulin were purchased from Invitrogen Life Technologies (A11001 and A11035).

### 1-2. Experimental animals

Mice were maintained in accordance with the policies of the CHA University Institutional Animal Care and Use Committee (IACUC). All the experiments were done to minimize animal suffering. Male C57Bl/6 mice were obtained from the Orient Bio (Seongnam, Korea) and maintained in the animal facility at CHA University Institutional Animal Care under a 12-h light/dark cycle with food and water *ad libitum*. This experiment was approved and conducted according to the regulations and guidelines of CHA University Institutional Animal Care and Use Committee.

### 1-3. Preparation and generation of liver organoids

Mouse LO had been developed previously. The mouse liver tissue was isolated and washed with ice-cold wash buffer (DMEM high glucose supplemented with 1% fetal bovine serum and 1% penicillin/streptomycin). Then, the tissue was incubated in the digestion buffer (0.125mg/ml dispase II and collagenase, 0.1mg/ml DNase I in wash medium) at 37°C for 40min under conditions of vigorous shaking. The ductal structures and other hepatocytes were obtained from the supernatant. The supernatant was collected and centrifuged at 200g for 10min. The pellets were mixed with Matrigel at a ratio of 1:1. The cells were embedded in organoid culture medium as described below. Advanced DMEM F/12 was supplemented with 10mM HEPES, 2mM Glutamax, 1mMN-acetylcysteine, 1x B-27, 5% R-spondin conditioned medium, 50ng/ml EGF, 100ng/ml noggin, 50ng/ml murine HGF, 200ng/ml FGF10, 10mM nicotinamide, and 10nM gastrin.

### 1-4. ECM-embedded organoid culture

Mouse LO were routinely maintained in organoid culture medium (Advanced DMEM F/12 was supplemented with 10mM HEPES, 2mM Glutamax, 1mMN-acetylcysteine, 1x B-27, 5% R-spondin conditioned medium, 50ng/ml EGF, 100ng/ml noggin, 50ng/ml murine HGF, 200ng/ml FGF10, 10mM nicotinamide, and 10nM gastrin). The organoids were passaged every 7 days; 1) mLOs were dissociated by mechanical trituration using a 1 ml syringe equipped with a 26G blunt needle. 2) The cells were centrifuged at 125 g for 5 min at 4°C and the supernatant was removed. 3) The cell pellet was resuspended in 100% Matrigel and cell suspension (25 µl) was dropped onto the center of a well in a prewarmed 48-well plate to form a dome-shape. 4) The plate was placed in a 37°C incubator for 1 hour to solidify the Matrigel. 5) Then, the Matrigel domes were immersed in the organoid culture medium, and the medium was changed every two or three days. 6) Olympus IX71S1F-3/DP70 inverted microscopy, EVOS XL core imaging system, and IncuCyte time-lapse microscopy system were utilized to observe organoid growth and morphology.

### 1-5. Floating culture

For floating culture, the organoid-embedding Matrigel (100%) was washed with PBS and pre-chilled Cell Recovery solution was added to the well at day 3. The Matrigel dome was disrupted by using gentle pipetting with wide bore 1000 p tip. Then, the organoids and Matrigel mixture was incubated on ice for approximately 30 min until the Matrigel matrix had been fully depolymerized. After incubation, the mixture was centrifuged at 125 g for 1 min. The pellet was washed 3 times with PBS containing 10% fetal bovine serum and suspended in organoid culture medium with 5% Matrigel. The organoids were transferred and incubated in an ultra-low attachment plate. The medium was replaced every two or three days.

### 1-6. Organoid differentiation

Mouse LO were seeded and kept in organoid culture medium for 3 days. Then the medium was changed to differentiation medium. The mouse liver differentiation medium was Advanced DMEM F/12 supplemented with 10mM HEPES, 2mM Glutamax, 1mMN-acetylcysteine, 1% penicillin/streptomycin, 1x B-27, 50ng/ml EGF, 100ng/ml FGF10, 10nM recombinant human [Leu15]-gastrin I, 50nM A83-01, and 10µM DAPT. The differentiation medium was freshly replaced each day for up to day 12. From day 13 to day 15, the organoids were incubated with the differentiation medium with 3 µM of dexamethasone.

### 1-7. Immunostaining

The organoids were fixed with 2% paraformaldehyde for 30min at room temperature. The fixed organoids were then blocked with 3% bovine serum albumin for 1 h after permeabilized with 0.2% Triton X-100 for 15 min. The organoids were incubated overnight at 4°C with the primary antibodies (1:50; HNF4α and 1:100; EpCAM) and treated with the fluorescence-conjugated secondary antibodies (1:1000; Alexa Fluor 488 and 546) for 2 h at room temperature in the dark. The organoids were washed 3 times with PBS for 10 min each after every step, and the nuclei were stained with DAPI (1:1000 diluted). The organoids were mounted on a confocal dish (coverslip-bottom culture dish) with PBS and observed with a confocal microscope (Olympus FV-3000) (Olympus, Tokyo, Japan).

### 1-8. Quantitative real-time polymerase chain reaction (qRT-PCR)

Liver organoids were extracted from Matrigel by pipetting and re-melted with Cell Recovery solution. The organoids and Matrigel mixture was centrifuged at 125 g for 3 min and the supernatant was removed. The pellet was washed 3 times with PBS containing 10% fetal bovine serum. RNA was extracted from organoids using TRIzol solution following manufacturer's instructions. RNA was transcribed to complementary DNA using PrimeScript 1st strand cDNA Synthesis Kit following manufacturer's instructions. Expression of selected genes was analyzed by using a real-time PCR system (QuantStudio 1) with the SYBR Green chemistry.

### 1-9. Quantification analysis

The images of organoids were obtained from IncuCyte time-lapse microscopy system. Individual organoids within the images were recognized and represented to purple boxes using OrgaQuant. To measure the diameter, the x and y coordinates from the bounding boxes of the detected organoids were transformed to meter and the area of individual organoids was calculated. Then, the graph of organoid size distribution was plotted between count and equivalent area diameter (*Dₐ*).

### 1-10. Nanoparticle cellular uptake assay; Fluorescence microscopy

Mouse LOs were exposed to 50 µg/ml of FITC-PS for 24 h. The organoids were fixed with 2% paraformaldehyde for 30min at room temperature. The nuclei were stained with DAPI (1:1000 diluted). The organoids were mounted on a confocal dish (coverslip-bottom culture dish) with PBS and observed with a confocal microscope (Olympus FV-3000).

For the paraffin section, the organoids were fixed with 2% paraformaldehyde for 30min at room temperature. The process for paraffin embedding schedule is as follows: 70% Ethanol for 30 minutes; 80% Ethanol for 30 minutes; 90% Ethanol for 30 minutes; 100% Ethanol for 30 minutes; Xylene for 1 hour, two times; Paraffin wax (60°C) for 2 hours on heat blocks. These processes are done in the 1.5 ml centrifuge tube. Then, cut at 5 mm from the bottom of the tube containing organoids. Put the tip of the tube on the embedding mold and melt paraffin wax. After melting the wax, move organoids into the embedding mold. Trim paraffin blocks and cut them at 10 µm. Serial sections can be obtained from an organoid. Mount sections on slides and bake it on a hotplate at 50°C overnight. Deparaffinization and hydration of sections is as follows: xylene for 5 minutes, two times; 100% ethanol for 3 minutes, two times; 95%, 80% and 70% ethanol for 1 minute each. Thereafter, the nuclei were stained with DAPI (1:1000 diluted). The coverslip was mounted with mounting medium on slides.

### 1-11. Nanoparticle cellular uptake assay; Flow cytometric analysis

Mouse LOs were exposed to 50 µg/ml of FITC-PS for 24 h. To obtain single cells from organoid, Matrigel was disrupted by pipetting and re-melted with Cell Recovery solution. The organoids and Matrigel mixture was centrifuged at 125 g for 3 min and the supernatant was removed. The pellet, which obtained organoids, was suspended and incubated with trypsin/EDTA at 37°C for 30 min. After incubation, the suspension was pipetted up/down with a 200 µl pipet tip. The pellet was washed 3 times with PBS containing 10% fetal bovine serum and re-suspended with the same buffer. Flow cytometric data were obtained using a CytoFLEX (Beckman Coulter, CA, USA) equipped with CytoExpert software (Beckman Coulter).

### 1-12. Nanoparticle Cellular uptake assay; Dark-field microscopy

The organoids were exposed to 20 µg/ml gold nanoparticles (50 nm) for 24 h. Nanoparticle cellular uptake image of organoids were acquired using a dark-field illumination. Two types of annulus beam, which is called a dark-field illumination, were used and generated by an enhanced dark-field illumination abbe condenser (Cytoviva) (AL, USA) and a reflective objective lens (LMPLFLN10XBD,Olympus) with a 10 mm working distance. The dark-field images captured by a CMOS camera (ORX-10GS-51S5C, Oryx, Teledyne) (CA, USA) with 2048 x 2048 pixel resolution and 3.45 µm pixel width. The magnification of dark-field image was 10x magnification using objective lens (RMS10X-PF, LMPLFLN10XBD, Olympus). For z-stack scanning, the nano z-positioning stage (MZS500-E Z-Axis Stage, Thorlabs) (NJ, USA) was used for the focus depth image of organoids. For time-lapse scanning, the organoids were captured every 10 sec for 1 h after AuNPs treatment.

### 1-13. Calcein/PI double stain assay

Mouse LOs were exposed to 20 µg/ml of gold nanoparticles (AuNPs) and 20 µg/ml of zinc oxide (ZnO) for 48 h. To detect live/dead cells, the organoids were stained with 1 µM of calcein-AM and 10 µg/ml of propidium iodide (PI) at 37°C for 30 min. After incubation, the organoids were washed 3 times with PBS and observed with a confocal microscope (Olympus FV-3000).

### 1-14. Apoptosis assay

Mouse LOs were exposed to 20 µg/ml of gold nanoparticles (AuNPs) and 20 µg/ml of zinc oxide (ZnO) for 48 h. Matrigel was disrupted by pipetting and re-melted with Cell Recovery solution. The organoids and Matrigel mixture was centrifuged at 125 g for 3 min and the supernatant was removed. The pellet, which obtained organoids, was suspended and incubated with trypsin/EDTA at 37°C for 30 min. After incubation, the suspension was pipetted up/down with a 200 µl pipet tip. The pellet was washed with PBS containing 10% fetal bovine serum and re-suspended in staining buffer containing fluorescein-5-isothiocyanate, ANXA5, and propidium iodide, provided in the apoptosis detection kit, according to the manufacturer's instructions. Flow cytometric data were obtained using a CytoFLEX (Beckman Coulter, CA, USA) equipped with CytoExpert software (Beckman Coulter).

### 1-15. Statistical analysis

Statistical analysis was performed in Sigma plot 15.0. Replicate data are expressed as the means ± standard deviation (S.D.). Significant differences between two non-parametric groups were statistically analyzed by Mann-Whitney U-test. One-way analysis of variance (ANOVA) was used for multiple comparisons. A p-value of <0.05 was considered statistically significant.

### Example 2. Examination of whether nanoparticles (NPs) penetrate ECM

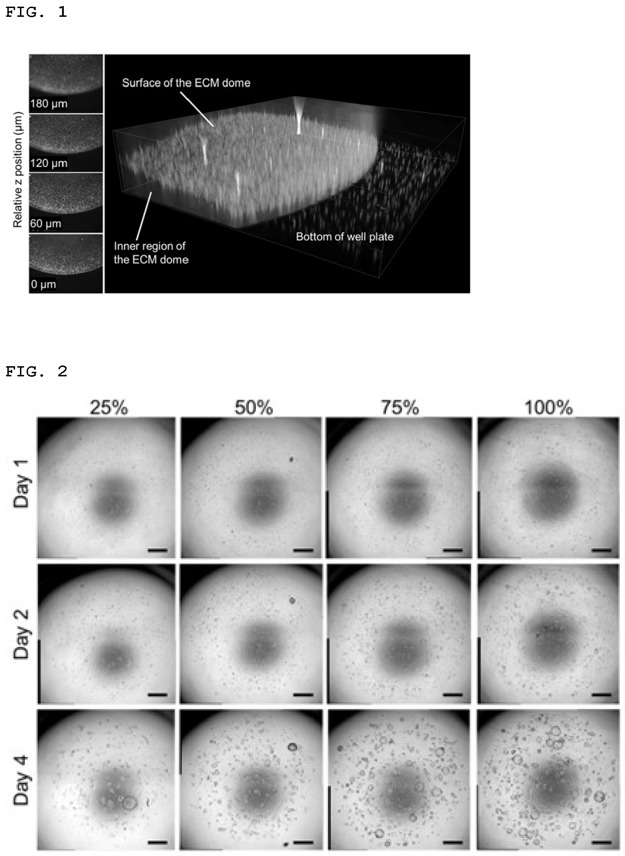

In the present invention, to examine whether nanoparticles penetrate existing organoids, whether nanoparticles (NPs) penetrate the ECM dome was examined. The ECM dome was exposed to AuNPs, which can be monitored using dark-field microscopy. The ECM dome was scanned with a 0.375 µm step size in the z-axis and 3D images were reconstructed from z-stack images (FIG. 1). 3D reconstruction revealed that AuNPs were located on the surface of the ECM dome and did not reach the inner region of the ECM dome. To test whether reducing the ECM concentration could improve the penetration of nanoparticles into cells, the present inventors cultured the organoid in domes containing different concentrations of ECM (FIG. 2). The present inventors selected mouse liver organoids (mLOs) because the liver, which is the main detoxification center in the human body, is where nanomaterials accumulate after entry through inhalation, ingestion, and dermal penetration.

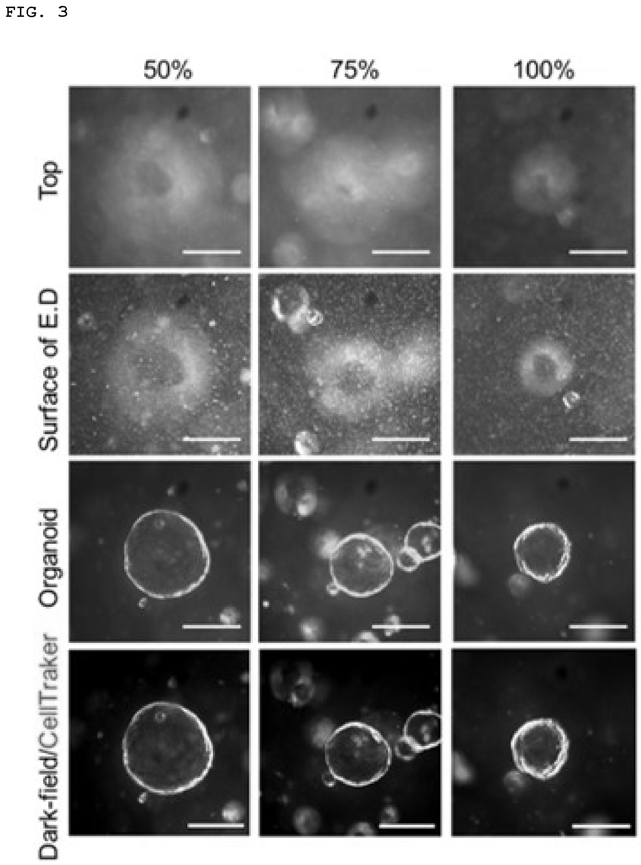

While spherical organoids were generated when high concentrations of ECM (>50%) were used in the domes, the cells did not form 3D structures and attached to the wells when a low concentration of ECM (25%) was used. The present inventors next investigated the cellular uptake of nanoparticles by cells in ECM-embedded organoids to confirm whether the ECM scaffold acts as a barrier to nanoparticles. Domes were prepared with various concentrations of ECM and the penetration of nanoparticles into the cells was measured (FIG. 3).

Mouse LOs were exposed to AuNPs, which can be monitored by using dark-field microscopy, for 24 h. Time-lapse images showed that AuNPs moved freely in the culture medium, whereas AuNPs were trapped and immobilized on the surface of the ECM dome, even at a 50% concentration of ECM. Because of the entrapment of AuNPs on the surface of the ECM dome, they were unable to penetrate the inner region of the ECM dome where the organoids were located. These results indicate that AuNPs did not penetrate the ECM dome and were not internalized by the cells (FIG. 3).

### Example 3. Establishment of cellular internalization system

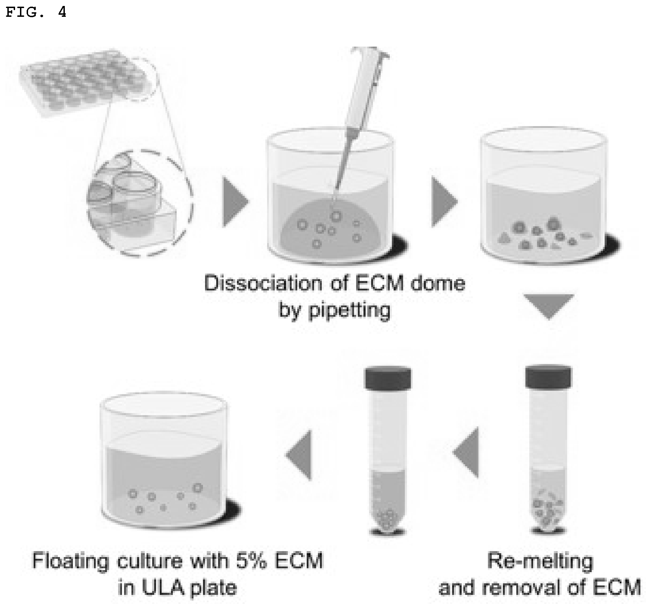

To overcome these limitations of the organoid culture system, the present inventors established a system in which mostly uniform organoids and efficient cellular internalization of nanoparticles can be obtained. A schematic illustration of this modified approach is shown in FIG. 4. Previous results (FIG. 1B) in the present invention showed that the organoid fragments were inevitably embedded and cultured within the dome when a high concentration of ECM (>50%) was used in the early step of the culture method, forming 3D structural organoids. Therefore, the present inventors designed a culture method for isolating mLOs from the ECM dome (100%) after organoid formation.

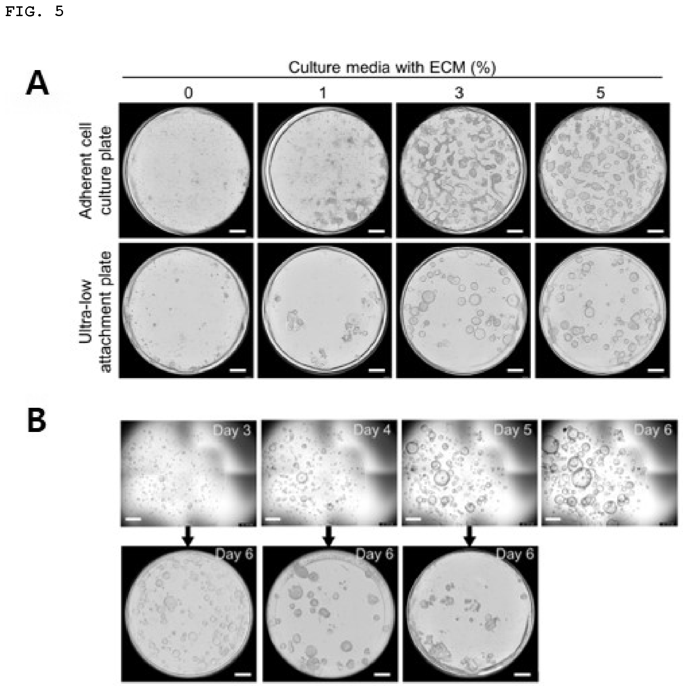

The ECM dome containing the formed mLOs was dissociated by gentle pipetting and re-melted using a cell recovery solution. The naked mLOs were then placed on an ultra-low-attachment plate with ECM-containing media. The optimal conditions for the floating culture system were determined to maintain the 3D spherical organoid structure (FIGS. 5A and 5B). After isolating the mLOs from the ECM dome, they were placed in different conditions, such as various types of culture plates and concentrations of ECM-supplemented media, and analyzed. When naked mLOs were transferred to a culture plate for adherent cells, the organoids showed the shape of a flat disk owing to intensive attachment to the well surface (FIG. 5A). In contrast, when the naked mLOs were transferred to an ultra-low attachment plate, they maintained their 3D spherical structure, except when a low concentration of ECM (1%)-supplemented culture media was used (FIG. 5A).

Next, the present inventors verified whether the timing of the transition of ECM-embedded organoids to a floating culture was important for maintaining their 3D structure (FIG. 5B). Since the interior of the organoid, which is organized into a 3D monolayer structure, is unfilled, organoids can be easily damaged by extracellular physical forces such as those that originate from pipetting and centrifugation. Thus, the naked mLOs isolated from the ECM dome were transitioned to a floating culture at different time points (3, 4, and 5 days). Mouse LOs transitioned on days 4 and 5 were observed to have ruptured and collapsed into 2D structures. In contrast, mLOs transitioned to a floating culture on day 3 showed the structural integrity of 3D spherical organoids. Considering all of the results, an ultra-low attachment plate with 5% ECM-containing media for the floating culture system and transition of the mLOs on day 3 were chosen as the optimal conditions to maintain the 3D spherical structure of the mLOs.

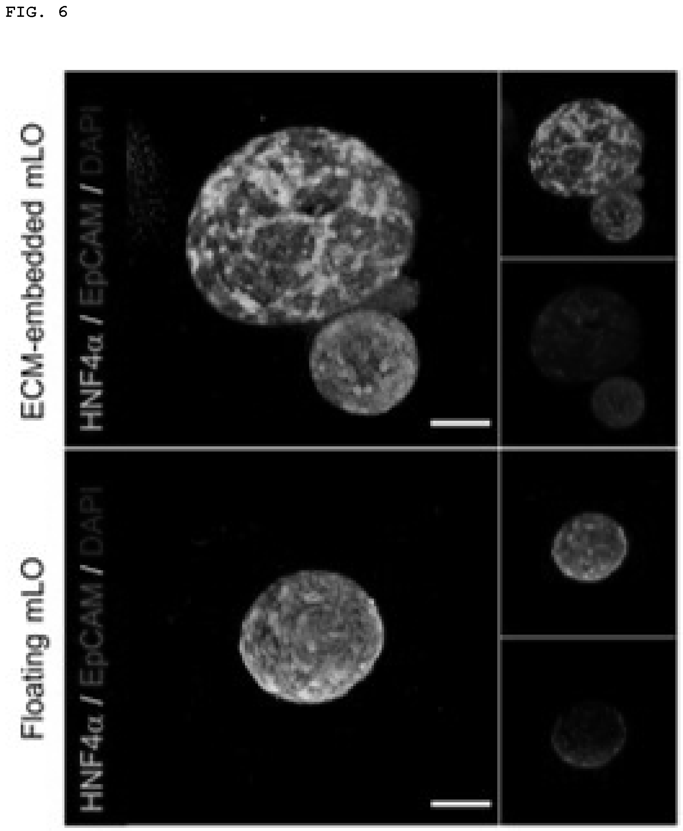

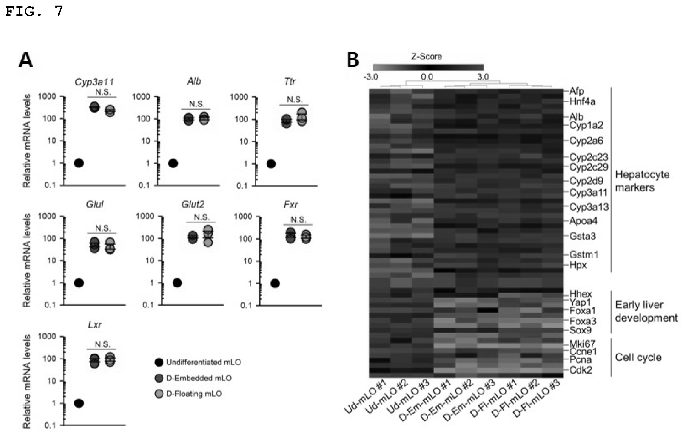

To investigate whether the mLOs in floating culture preserved the hepatic differentiation capacity, the present inventors analyzed gene or protein expression levels of characteristic hepatic markers (FIGS. 6 and 7A). Immunostaining revealed the expression of the hepatocyte markers HNF-4α and EpCAM in the differentiated mLOs cultured using both ECM-embedded and floating systems (FIG. 6). Consistent with this result, gene expression analyzes showed the substantial upregulation of hepatocyte markers in both ECM-embedded mLOs and floating mLOs after differentiation (FIGS. 7A and 7B).

These results indicate that the organoids in floating culture exhibit hepatocyte-like characteristics and maintain a 3D spherical structure without the ECM-scaffold.

### Example 4. Identification of individual organoids

In general, organoids cultured in ECM domes show size heterogeneity; organoids in the core of the dome tend to be smaller than those at the edge of the dome. This size heterogeneity of organoids, which are embedded and cultured in an ECM dome, is induced by the spatiotemporal concentration gradient of morphogens (especially Wnt3a), oxygen, and nutrients. This is because the edge of the dome, where the ECM is thin, has a smooth supply of morphogens, oxygen, and nutrients. However, the mLOs in floating culture, transitioned on day 3 and evaluated on day 6, showed relatively uniform size (FIGS. 5A and 5B). The present inventors speculated that the floating culture method has relatively low size heterogeneity because morphogens, oxygen, and nutrients are supplied to all organoids at similar levels.

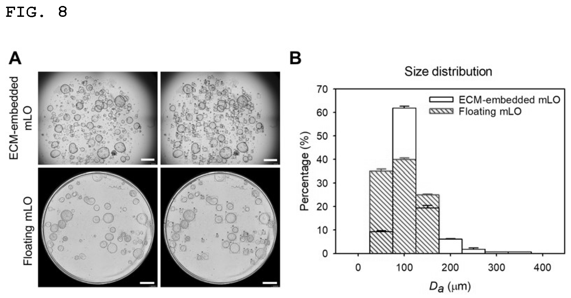

To measure and quantify the size of the organoids, OrgaQuant software was used to identify individual organoids in images recorded on day 6 (see the purple boxes in FIG. 8A). The size distribution of the organoids in the floating culture system showed a narrower peak than that of the ECM-embedded organoids (FIG. 8B). These results indicate that the floating culture system forms organoids of a more uniform size than the ECM-embedded organoid system.

### Example 5. Examination of the efficiency of internalization of nanoparticles in floating culture organoids

Next, the present inventors examined the efficiency of internalization of nanoparticles in both ECM-embedded and floating culture organoids to determine whether the floating culture system could be used to assess the safety of nanomaterials.

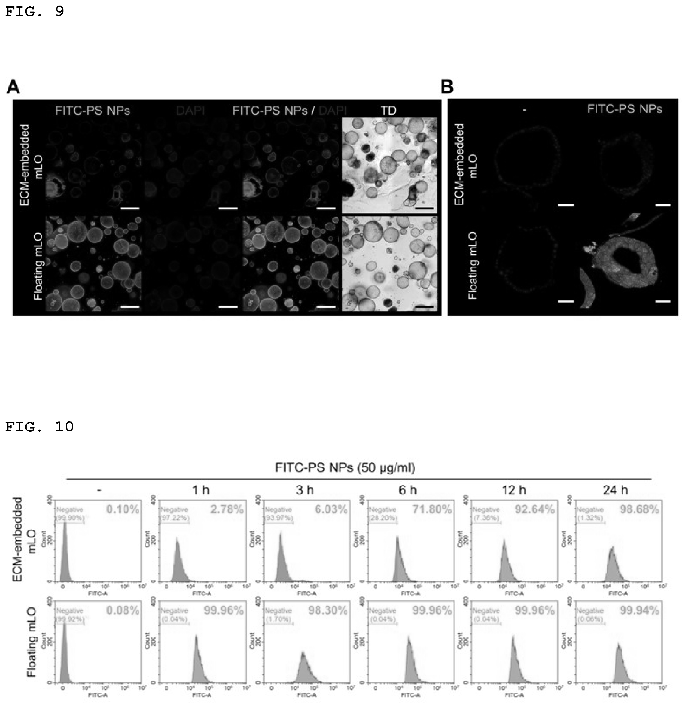

To monitor the cellular internalization of nanoparticles, mLOs were exposed to green fluorescent FITC-labeled polystyrene nanoparticles (FITC-PS NPs, 50 nm) (FIGS. 9A, 9B and 10). The floating culture organoids showed a significantly stronger FITC fluorescence signal than that of ECM-embedded organoids. The cells were further analyzed to quantify the FITC-PS NPs-positive cells using flow cytometry; organoids were exposed to FITC-PS NPs and then separated into single cells using trypsin/EDTA.

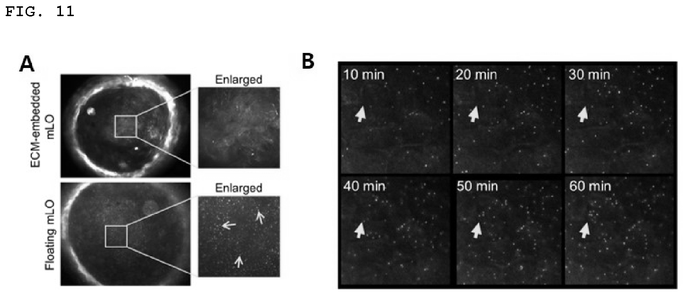

The population of FITC-positive cells after 1 h of FITC-PS NPs incubation was >99.9% in the floating culture organoids and only 2.78% in the ECM-embedded organoids, increasing to >70% after 6 h of exposure. Next, the present inventors examined the cellular uptake of AuNPs by scanning the whole organoid using dark-field microscopy (FIGS. 11A and 11B). Similar to the preceding results (FIGS. 9A, 9B and 10), AuNPs were embedded at the top of the ECM dome and did not internalize within the cells. However, AuNPs were observed inside the cells in floating culture organoids as detected using time-lapse scanning (FIG. 11). These results indicated that the floating culture method is more conducive to the internalization of nanoparticles than is the ECM-embedded organoid culture method.

### Example 6. Evaluation of organoid-based toxicity of nanoparticles

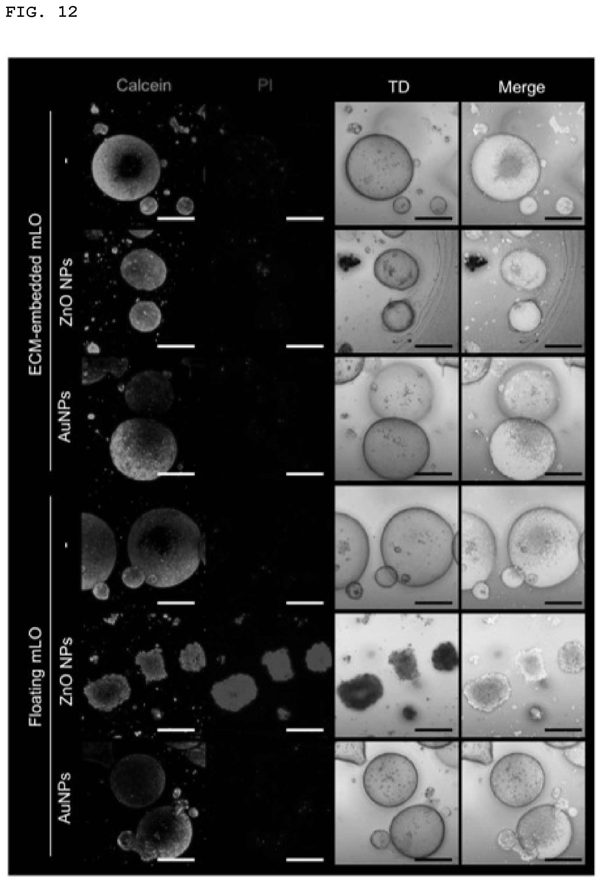

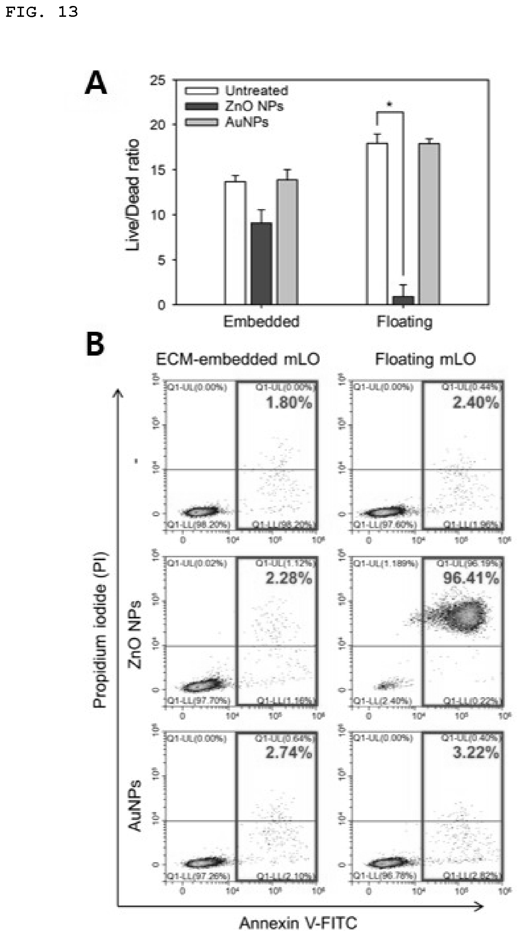

Finally, to evaluate any organoid-based toxicity of the nanoparticles, the present inventors performed a live/dead cell assay and apoptosis assay of mLOs after exposure to nanoparticles (FIGS. 12 and 13). Gold nanoparticles (AuNPs) and zinc oxide nanoparticles (ZnO NPs), which are widely used in many different fields, were selected for testing the organoid-based toxicity experiment. It depends on the properties such as size, shape, charging, and presence of ligand, but numerous studies reported that AuNPs did not cause acute toxicity. The present inventors selected spherical AuNPs, which were reported to be nontoxic both *in vitro* and *in vivo*, as negative controls.

Meanwhile, ZnO NPs have been known to induce toxicity in various kinds of cells *in vitro* and *in vivo*. Thus, ZnO NPs were selected to serve as positive controls for cytotoxicity. Organoids were exposed to ZnO NPs and AuNPs for 48 h, and cytotoxicity was measured using calcein and propidium iodide (PI) staining (FIGS. 12 and 13A). In both the ECM-embedded and floating culture organoids not exposed to nanoparticles, a comparatively weak red fluorescence signal was observed, which represented dead cells. When the organoids were exposed to ZnO NPs, the red fluorescence signal significantly increased in the floating culture organoids, whereas the signal did not change in the ECM-embedded organoids compared to that of background levels. In contrast, when the organoids were exposed to AuNPs, a weak red fluorescence signal was observed in both ECM-embedded and floating culture organoids. That is, it can be seen that cytotoxicity was observed only in floating culture organoids exposed to ZnO NPs. To further examine NP-induced apoptotic cell death, the present inventors monitored apoptosis using flow cytometry (FIG. 13B).

AuNPs did not increase the apoptotic cell death in ECM-embedded or floating organoids. However, when the organoids were exposed to ZnO NPs, the population of apoptotic cells significantly increased in the floating organoids, but not in the ECM-embedded organoids. The reason why cytotoxicity varied depending on the cell culture method despite exposure to the same concentration of ZnO NPs appears to be due to internalization of the nanoparticles. Previous results in the present invention suggested that the nanoparticles could only be internalized in floating culture organoids and not in ECM-embedded organoids; thus, in live/dead cell assays and apoptosis assays, reliable results can only be obtained using floating culture organoids. ECM-embedded organoids did not show significant cytotoxicity even upon exposure to ZnO NPs or AuNPs. This result can cause misunderstanding that both nanoparticles are safe regardless of the presence or absence of nanoparticles owing to the poor penetration of the nanoparticles. Collectively, the results of these studies suggest that a floating culture organoid system is a promising tool for assessing the safety and toxicity of nanomaterials.

In conclusion, the present inventors developed a new method for culturing organoids that may be used to assess the safety and toxicity of nanomaterials. Specifically, organoids released from the ECM and grown in floating culture in an ultra-low attachment plate exhibited 3D spherical structures of relatively uniform size while maintaining differentiation potential equivalent to that of conventional ECM-based culture methods. Moreover, the floating culture method is more conducive to the internalization of nanoparticles. In turn, this facilitates the utility of the system for assessing nanoparticle cytotoxicity, as shown by the insensitivity of ECM-embedded organoids to ZnO NPs exposure that causes cell death in floating organoids.

Thus, the floating culture system provides a reliable tool for assessing the safety of nanomaterials and has the potential to be utilized in high-throughput nano-safety screening. This safety assessment system allows for acceleration in research and development of nanomaterials in various fields by ensuring toxicity monitoring and the safe use of nanomaterials.

So far, the present invention has been described with reference to the embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

### Industrial Applicability

The present invention relates to a method for floating culture of liver organoids for assessing the safety and toxicity of nanomaterials. When the floating culture system of the present invention is used, it provides a reliable tool for assessing the safety of nanomaterials, has the potential to be utilized in high-throughput nano-safety screening, and allows for acceleration in research and development of nanomaterials in various fields. Thus, the present invention is industrially applicable.

## Claims

1. A cell floating culture method for assessing nanomaterial toxicity, comprising:
step (a) of preparing organoids from adult stem cells, embryonic stem cells, or induced pluripotent stem cells;
step (b) of embedding the prepared organoids in an extracellular matrix (ECM), followed by culture;
step (c) of resuspending the organoids, embedded in the ECM, in Matrigel;
step (d) of washing the Matrigel containing the organoids, followed by disruption to isolate only the organoids;
step (e) of suspending the isolated organoids in an organoid culture medium containing Matrigel at a lower concentration than that of the Matrigel used in step (c);
step (f) of incubating the suspended organoids in an ultra-low attachment plate; and
step (g) of differentiating the incubated organoids.

2. The cell floating culture method of claim 1, wherein the organoids mimic a function of at least one selected from the group consisting of cerebrum, gastrointestinal tract, small intestine, large intestine, stomach, lingua, thyroid, thymus, testis, prostate, liver, pancreas, epithelium, kidney, heart, retina, and glioblastoma.

3. The cell floating culture method of claim 1, wherein the ECM used in step (b) has a concentration of 50 to 100%.

4. The cell floating culture method of claim 1, wherein the Matrigel contained in the organoid culture medium used in step (e) has a concentration of 1 to 10%.

5. The cell floating culture method of claim 1, wherein the medium in the incubation in step (f) is replaced every 1 to 5 days.

6. The cell floating culture method of claim 1, wherein step (g) of differentiating the organoids comprises:
step (1) of replacing the medium of the organoids subjected to floating culture with a differentiation medium;
step (2) of replacing the differentiation medium daily; and
step (3) of adding an anti-inflammatory agent to the differentiation medium.

7. The cell floating culture method of claim 6, wherein the differentiation medium in step (2) is replaced daily for 10 to 15 days.

8. The cell floating culture method of claim 6, wherein the anti-inflammatory agent in step (3) is at least one selected from the group consisting of steroids, non-steroids, immune selective anti-inflammatory derivatives, herbal extracts, and antioxidants.

9. The cell floating culture method of claim 6, wherein the anti-inflammatory agent in step (3) is added for 1 to 5 days.

10. A floating culture organoid for assessing nanomaterial toxicity prepared by the method of claim 1.

11. A kit for evaluating nanomaterial toxicity comprising the floating culture organoid of claim 10.
